# EUROPEAN PATENT SPECIFICATION

(11) **EP 2 608 833 B1**
(45) Date of publication and mention of the grant of the patent: **19.07.2017**
(21) Application number: 11749525.9
(22) Date of filing: 19.07.2011
(51) Int. Cl.: A61M 16/06

(54) **CUSHION FOR A PATIENT INTERFACE DEVICE**
KISSEN FÜR EINE PATIENTENSCHNITTSTELLE
COUSSIN POUR UN DISPOSITIF D'INTERFACE PATIENT

(30) Priority: 27.08.2010 US 377698 P
(43) Date of publication of application: 03.07.2013
(73) Proprietor: Koninklijke Philips N.V., 5656 AE Eindhoven (NL)
(72) Inventor: HO, Peter Chi Fai, 5656 AE Eindhoven (NL); EURY, Matthew Paul, 5656 AE Eindhoven (NL); MATULA, Jerome Jr., 5656 AE Eindhoven (NL)
(74) Representative: Steffen, Thomas
(86) International application number: PCT/IB2011/053206
(87) International publication number: WO 2012/025843

(56) References cited:
- WO-A1-01/62326
- WO-A1-2007/068044
- WO-A1-2009/062265
- US-A1- 2006 096 598
- US-A1- 2007 163 594

## Description

### BACKGROUND OF THE INVENTION

### 1. Field of the Invention

The present invention relates to patient interface devices for communicating a flow of gas with an airway of a user, and, in particular, to a cushion having multiple voids and/or orifices for providing controlled deformation and collapse of the cushion.

### 2. Description of the Related Art

There are numerous situations where it is necessary or desirable to deliver a flow of breathing gas non-invasively to the airway of a patient, i.e., without intubating the patient or surgically inserting a tracheal tube in their esophagus. For example, it is known to ventilate a patient using a technique known as non-invasive ventilation. It is also known to deliver positive airway pressure (PAP) therapy to treat certain medical disorders, the most notable of which is obstructive sleep apnea (OSA). Known PAP therapies include continuous positive airway pressure (CPAP), wherein a constant positive pressure is provided to the airway of the patient in order to splint open the patient's airway, and variable airway pressure, wherein the pressure provided to the airway of the patient is varied with the patient's respiratory cycle. Such therapies are typically provided to the patient at night while the patient is sleeping.

Non-invasive ventilation and pressure support therapies as just described involve the placement of a patient interface device including a mask component having a soft, flexible cushion on the face of a patient. The mask component may be, without limitation, a nasal mask that covers the patient's nose, a nasal cushion having nasal prongs that are received within the patient's nares, a nasal/oral mask that covers the nose and mouth, or a full face mask that covers the patient's face. Such patient interface devices may also employ other patient contacting components, such as forehead supports, cheek pads and chin pads. The patient interface device interfaces the ventilator or pressure support device with the airway of the patient, so that a flow of breathing gas can be delivered from the pressure/flow generating device to the airway of the patient. It is known to maintain such devices on the face of a wearer by a headgear having one or more straps adapted to fit over/around the patient's head.

Current mask cushions are of a singular or multiple, thin curved flap design in silicone. The majority of such known cushions have a unitary top flap for sealing and a partial, thicker inner section for support. These common, unitary flaps result in uniform deformation. Such uniform deformation does not, however, always provide an optimal seal as certain portions of the patient's face may optimally need more or less deformation than is provided.

Exemplary mask cushions are known from WO 2009/062265 A1, US 2007/163594 A1, WO 2007/068044 A1 WO 01/62326 A1 and from US 2006/096598 A1.

### SUMMARY OF THE INVENTION

Accordingly, it is an object of the present invention to provide a cushion for a patient interface device that overcomes the shortcomings of conventional cushion.

This object is achieved by a cushion for a patient interface device according to claim 1 that includes a patient contacting portion, a connection portion configured to provide a secure connection between the cushion and a support structure, and a middle portion provided in between the patient contacting portion and the connection portion, the middle portion including one or more wall portions adjacent the patient contacting portion. The wall portions include a plurality of voids defined therein. The patient contacting portion and the one or more wall portions are made of a material having a hardness of between 5 and 60 on the Shore 00 scale. The plurality of voids comprise at least one first void provided in an inner side of the one or more wall portions and at least one second void provided in an outer side of the one or more wall portions, wherein the parts of the one or more wall portions not having the plurality of voids provided therein all have a cross-sectional thickness of between 4 mm and 10 mm, wherein the one or more wall portions comprise an apex wall, a left side wall, a right side wall and a bottom wall, wherein the plurality of voids comprise: (i) a first void provided in an inner side of the apex wall, the left side wall, the right side wall and the bottom wall, (ii) a second void provided in an outer side of the apex wall, the left side wall, and the right side wall, (iii) a third void provided in the outer side of the left side wall below the second void, (iv) a fourth void provided in the outer side of the right side wall below the second void, (v) a fifth void provided in the outer side of the left side wall at least partially above the third void and below the second void, (vi) a sixth void provided in the outer side of the right side wall at least partially above the fourth void below the second void, and (vii) a seventh void provided in the outer side of the bottom wall, wherein the plurality of voids are recessed portions within the exterior surface on the inner and outer side of the one or more wall portions.

In another aspect, there is disclosed a cushion for a patient interface device that includes a patient contacting portion, a connection portion or providing a connection between the cushion and a support structure, and a middle portion located in between the patient contacting portion and the connection portion, the middle portion having one or more wall portions, wherein the one or more wall portions include a plurality of voids therein, wherein the parts of the one or more wall portions not having the plurality of voids provided therein have a cross-sectional thickness of 4 mm or greater.

In still another aspect, a cushion for a patient interface device is provided that includes a patient contacting portion and a middle portion located adjacent to the patient contacting portion, the middle portion having one or more wall portions, wherein the one or more wall portions include a plurality of voids therein,
wherein the plurality of voids comprise a first void provided in an inner side of a first one of the wall portions and a second void provided in an outer side of the first one of the wall portions, wherein in a cross-section of the first one of the wall portions the first void and the second void are positioned across from and overlapping one another such that at least a portion of the first void and a portion of the second void are located the same distance from a plane perpendicular to the cross-section and defining a bottom of the middle portion with a section of the middle portion being located between the first void and the second void.

In yet another embodiment, a patient interface device including a support structure, such as a shell or faceplate, and a fluid coupling conduit, such as an elbow connector, is provided wherein any of the cushion embodiments just described is coupled to the support structure. In another embodiment, a patient sealing assembly for a patient interface device is provided that includes a cushion element having one or more wall portions, wherein the one or more wall portions include one or more orifices extending completely therethrough, and a sealing flap assembly structured to be coupled to the cushion element, the sealing flap assembly having a sealing flap portion structured to cover the one or more orifices when the sealing flap assembly is coupled to the cushion element.

In still another aspect, a cushion for a patient interface device that includes a sealing flap, a connection portion for providing a connection between the cushion and a support structure, and a wall portion located in between the sealing flap and the connection portion, wherein the wall portion includes a plurality of voids therein, wherein the parts of the wall portion not having the plurality of voids provided therein have a cross-sectional thickness of 3 mm or greater and the wherein the sealing flap has a cross-sectional thickness of between 0.1 mm and 0.5 mm.

In yet another aspect, a cushion for a patient interface device is provided that includes a sealing flap, a connection portion for providing a connection between the cushion and a support structure, and a wall portion located in between the sealing flap and the connection portion, wherein the wall portion includes a plurality of voids therein, wherein the parts of the wall portion not having the plurality of voids provided therein have a cross-sectional thickness T, and wherein the parts of the wall portion having the plurality of voids provided therein have a cross-sectional thickness of between 5% and 9% of T.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

### BRIEF DESCRIPTION OF THE DRAWINGS

FIG. 1 is a front perspective view, FIG. 2 is a rear perspective view, and FIG. 3 is a side view of a system adapted to provide a regimen of respiratory therapy to a patient according to one exemplary embodiment of the present invention;
FIG. 4 is a front perspective view, FIG. 5 is a rear perspective view and FIG. 6 is a side view of a cushion according to an exemplary embodiment of the present invention forming a part of a patent interface device of the system of FIGS. 1-3;
FIGS. 7-11 are various cross-sectional views of the cushion of FIGS. 4-6;
FIG. 12 is a cross sectional view of a cushion according to an alternative embodiment of the present invention;
FIGS. 13 and 14 are front and rear perspective views, respectively, of a system adapted to provide a regimen of respiratory therapy to a patient according to an alternative exemplary embodiment of the present invention;
FIG. 15 is a front perspective view and FIG. 16 is a rear perspective view of a cushion assembly according to an alternative exemplary embodiment of the present invention forming a part of a patent interface device of the system of FIGS. 13 and 14;
FIGS. 17-19 are various cross-sectional views of the cushion of FIGS. 15 and 16;
FIG. 20 is a side view and FIG. 21 is a front perspective view of a cushion element forming a part of a patient sealing element according an alternative exemplary embodiment of the present invention;
FIG. 22 is a side view of a sealing flap assembly which together with the cushion element of FIGS. 20 and 21 form a patient sealing assembly according an alternative exemplary embodiment of the present invention;
FIGS. 23A-23G are side views of alternative cushion elements wherein the orifices defined therein have alternative shapes; and
FIG. 24 is a side view of a cushion according a further alternative exemplary embodiment of the present invention.

### DETAILED DESCRIPTION OF THE EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

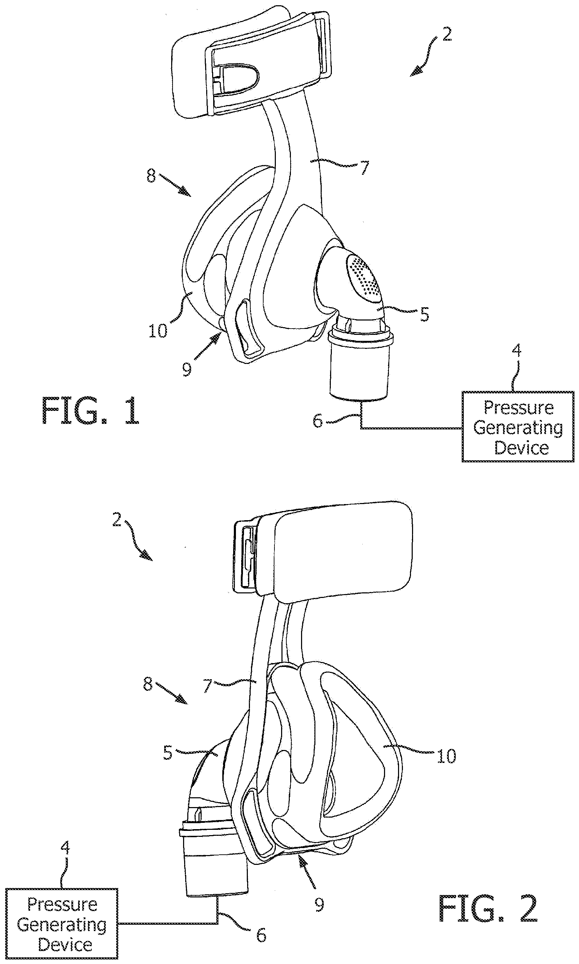

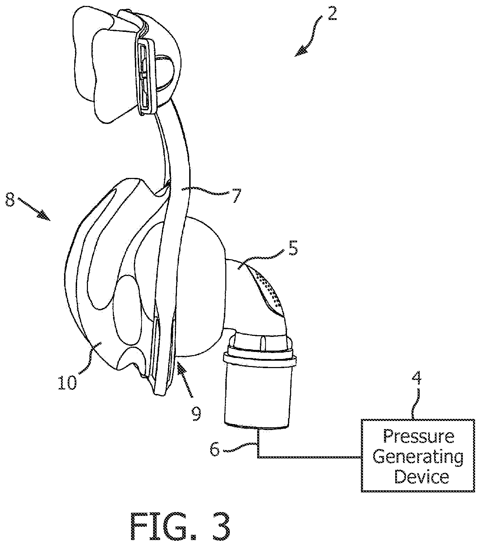

FIGS. 1, 2, and 3 are front perspective, rear perspective and side views, respectively, of system 2 adapted to provide a regimen of respiratory therapy to a patient with a patient interface device according to one exemplary embodiment of the present invention. System 2 includes pressure generating device 4, delivery conduit 6, and patient interface device 8. Pressure generating device 4 is structured to generate a flow of breathing gas and may include, without limitation, ventilators, constant pressure support devices (such as a continuous positive airway pressure device, or CPAP device), variable pressure devices (e.g., BiPAP®, Bi-Flex®, or C-Flex™ devices manufactured and distributed by Philips Respironics of Murrysville, Pennsylvania), and auto-titration pressure support devices.

Delivery conduit 6 is structured to communicate the flow of breathing gas from pressure generating device 4 to a patient interface device 8. Patient interface device 8, which in the illustrated embodiment is a nasal mask 9. However, any type of mask, such as a nasal/oral mask, a nasal cushion (e.g., including nasal prongs) or a full face mask, which facilitates the delivery of a flow of breathing gas to the airway of a patient, may be used as mask 9 while remaining within the scope of the present invention. Mask 9 includes a sealing cushion 10, which is fluidly coupled to a rigid support structure 7. Support structure 7 is fluidly coupled to an elbow conduit 5. Elbow conduit 5 is structured to be coupled to delivery conduit 6, which is in fluid communication with pressure generating device 4. As seen in FIGS. 1-3, support structure 7 is structured to be coupled to a headgear assembly (not shown) for securing patient interface device 8 to the head of the patient.

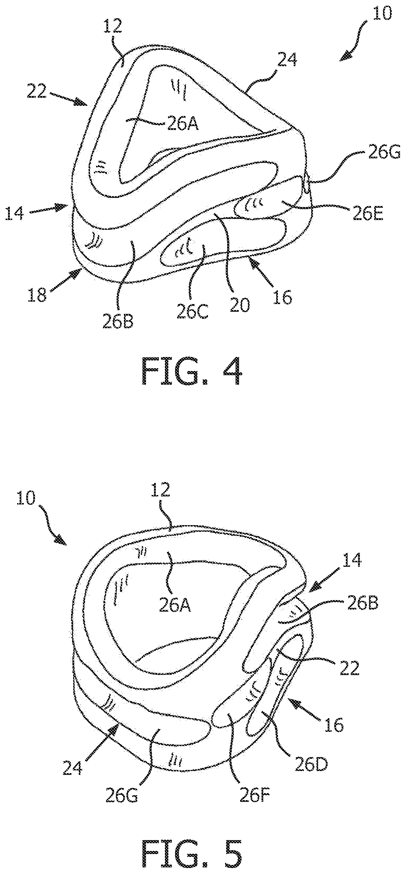

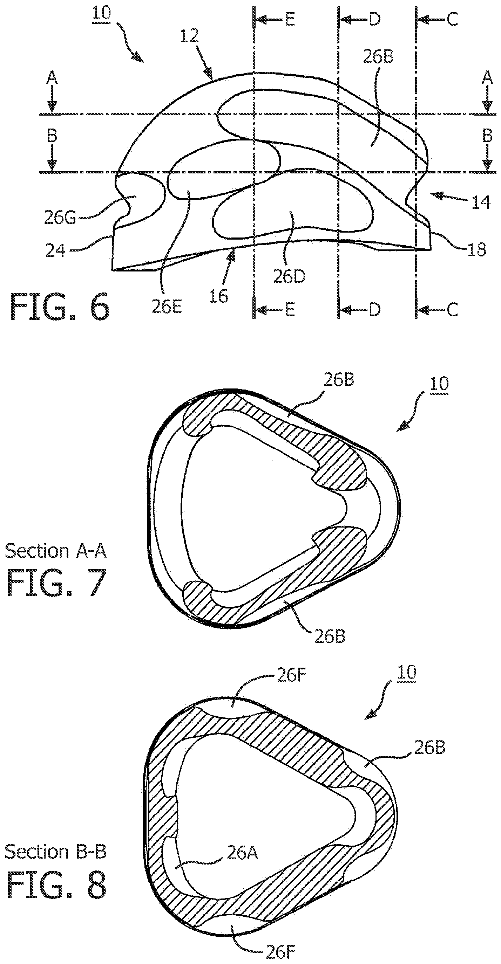

FIG. 4 is a front perspective view, FIG. 5 is a rear perspective view, and FIG. 6 is a side view of cushion 10 according to an exemplary embodiment of the present invention. As seen in FIGS. 4, 5 and 6, cushion 10 has a generally triangular-shaped periphery and is structured to fit over the patient's nose. However, it is well known in the art that various patient interfaces can be constructed which cover the patient's nose and mouth (nasal-oral), cover a majority of the patient's face (full-face mask), or fit within the patient's nares. The present invention contemplates that the unique aspects of this invention could be utilized on a variety of different patient interfaces without departing from the scope of this invention. Cushion 10 is configured to provide a flexible seal between the patient and patient interface device 8.

Cushion 10 has a patient contacting portion 12, a middle portion 14, and a connection portion 16. Each portion is specifically configured to achieve different goals. Patient contacting portion 12 is configured to provide a secure seal between the user (the patient) and patient interface device 8. Middle portion 14 is configured to provide support to cushion 10, and, as described in greater detail below, to provide for a controlled collapse, deformation and rebound of cushion 10 in response to forces exerted on cushion 10. Connection portion 16 is configured to provide a secure connection between cushion 10 and support structure 7. Furthermore, as noted above, cushion 10 has a generally triangular-shaped periphery and thus middle portion 14 includes apex wall 18, left side wall 20, right side wall 22 and bottom wall 24.

In addition, as seen in FIGS. 4-6, middle portion 14 of cushion 10 includes a plurality of voids 26 provided in the inner side and/or outer side of one or more of apex wall 18, left side wall 20, right side wall 22 and bottom wall 24. As used herein, the term "void" shall refer to an inwardly extending (toward the center of the wall) or recessed portion of a wall of cushion 10 defined and surrounded by edge portions of the exterior surface (on the inner or outer side) of the wall.

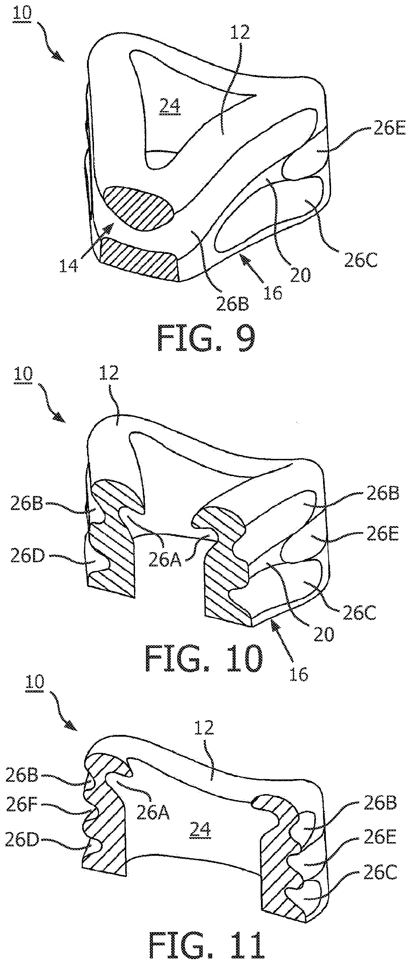

In the exemplary embodiment of cushion 10 shown in FIGS. 4-6, cushion 10 includes seven voids 26. FIGS. 7-11 are various cross-sectional views of cushion 10 that help to show the various voids 26 of the illustrated embodiment. FIG. 7 is taken along lines A-A of FIG. 6, FIG. 8 is taken along lines B-B of FIG. 6, FIG. 9 is taken along lines C-C of FIG. 6, FIG. 10 is taken along lines D-D of FIG. 6, and FIG. 11 is taken along lines E-E of FIG. 6. As seen in FIGS. 4-11, those seven voids 26 include: (i) a first void 26A provided in the inner side (at the top thereof) of apex wall 18, left side wall 20, right side wall 22 and bottom wall 24 (void 26A thus extends continually around the entire inner top periphery of cushion 10), (ii) a second void 26B provided in the outer side of apex wall 18, left side wall 20 (at the top thereof), and right side wall 22 (at the top thereof) (void 26B thus extends continuously from the rear end of left side wall 20 to the rear end of right side wall 22), (iii) a third void 26C provided in the outer side of left side wall 20 (at the bottom thereof), (iv) a fourth void 26D provided in the outer side of right side wall 22 (at the bottom thereof), (v) a fifth void 26E provided in the outer side of left side wall 20 (at the rear, middle portion thereof), (vi) a sixth void 26F provided in the outer side of right side wall 22 (at the rear, middle portion thereof), and (vii) a seventh void 26G provided in the outer side of bottom wall 24.

In one particular, non-limiting exemplary embodiment of cushion 10, the dimensions of voids 26A-26G of cushion 10 are as follows. The depth dimensions provided are the proximal maximum depth or range of depths of the voids 26 and all dimensions are in mm. It should be understood that the values given are only some typical values which may change according to the compression force. Front is toward the tip of the triangular shape while base is the bottom. The width (W) of each void varies, and this width values gives are the proximal widths. Void 26A ranges in depth from 3.5 to 10 mm (from inner wall - extends all way round, W=12 mm), void 26B ranges in depth from 4 to 5 mm (from outer wall - extends side to front 100 mm, W=8 mm), Voids 26C and 26D are 7 mm in depth (from outer wall - extends along lower side 32 mm, W=9 mm), void 26E and 26 F is 5.5 mm (from outer wall - extends along side 22 mm, W=8.5 mm), and void 26G is 3.2 mm (from outer wall - extends along base is 50, W=8.9).

In addition, in one exemplary non-limiting embodiment, cushion 10 is defined from a unitary piece of soft, flexible, cushiony, elastomeric material, such as, without limitation, a single component silicone rubber (RTV-1), a two component silicone (RTV-2), an appropriately soft thermoplastic elastomer, a foam, or any combination of such materials. In an exemplary embodiment, the material has a hardness of between 5 and 60 on the Shore 00 scale. This material is much softer than materials that are used in current, prior art cushions, wherein the materials have a hardness of around 40 Shore A.

In another exemplary non-limiting embodiment, which may or may not also use the material having the hardness just described, the portions of apex wall 18, left side wall 20, right side wall 22 and bottom wall 24 of middle portion 14 that do not include a void 26 have a cross-sectional thickness (shown by, for example, the arrows in FIGS. 10 and 11) of 4 mm or greater. In an alternative exemplary embodiment, that cross-sectional thickness is 5 mm or greater, and in still another alternative exemplary embodiment, that cross-sectional thickness is between 4 mm and 10 mm or between 5 mm and 10 mm. These thicknesses are significantly greater than the wall thickness of current, prior art cushions, which typically have a thickness of between 0.3 and 1.5 mm.

Furthermore, as seen in FIGS. 10 and 11, in the illustrated embodiment, one or more cross-sectional portions of apex wall 18, left side wall 20, right side wall 22 and/or bottom wall 24 of middle portion 14 have a first void 26 on the outer side thereof and a second void 26 on the inner side thereof which are positioned so that they are aligned across from one another such that at least a portion of the first void 26 overlaps a portion of the second void 26 (with a section of the middle portion 14 being located therebetween). In other words, the overlapping first and second voids 26 are positioned such that at least a portion of the first void 26 and a portion of the second void 26 are located the same distance from a plane defining a bottom of middle portion 14. One example of such overlapping first and second voids 26 are voids 26A and 26B shown in FIGS. 10 and 11. In one particular, non-limiting embodiment, the amount of overlap between the voids 26 is 2 mm or more.

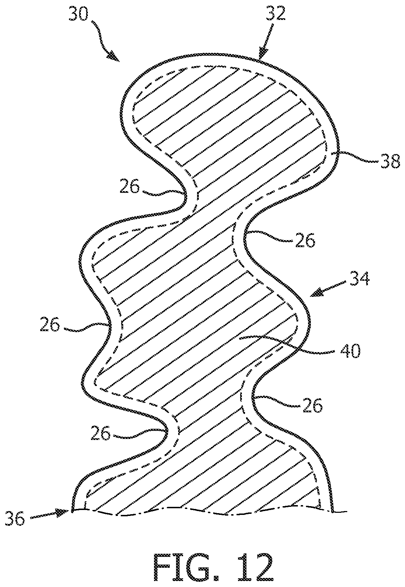

FIG. 12 is a cross sectional view of cushion 30 according to an alternative embodiment of the present invention. As seen in FIG. 10, cushion 30 includes a patient contacting portion 32, a middle portion 34 having a plurality of voids 26 provided therein, and a connection portion 36. As seen in FIG. 10, cushion 30 is constructed from a hollow formed bladder 38, which defines the outer shape of cushion 30, which is filled with a soft, cushiony material 40, such as, without limitation, gel, fluid or foam. Bladder 38 may be made of an elastic material, such as, without limitation, silicone, TPE or slush-molded rubber. In one particular embodiment, material 40 has a hardness of between 5 and 60 on the Shore 00 scale. Bladder 38 is formed from a harder material, for example 10 to 40 shore A Liquid Silicone Rubber, TPE, or slush-molded rubber.

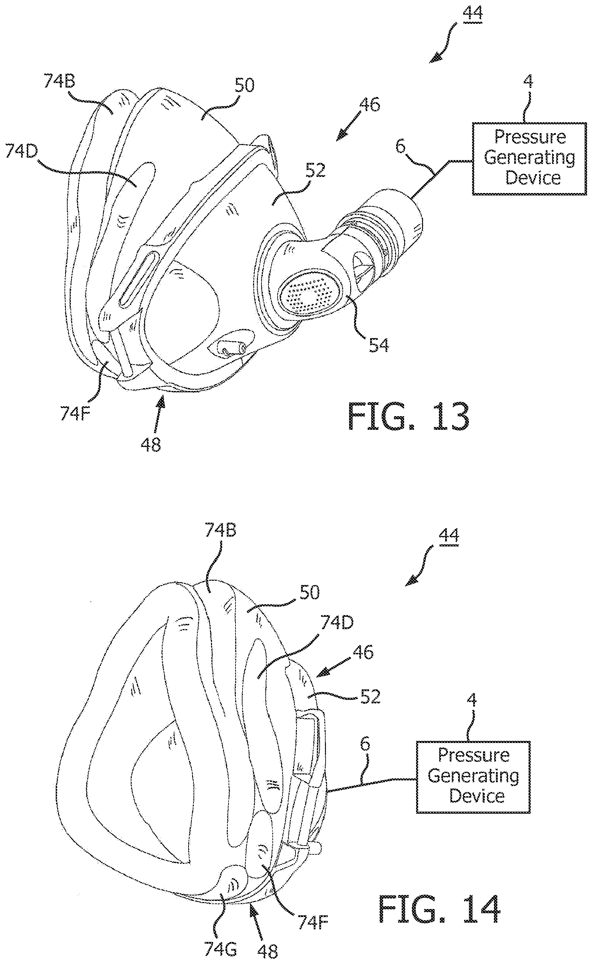

FIGS. 13 and 14 are front and rear perspective/schematic views, respectively, of system 44 adapted to provide a regimen of respiratory therapy to a patient according to an alternative exemplary embodiment of the present invention. System 44 includes pressure generating device 4 and delivery conduit 6 as described elsewhere herein. System 44 also includes alternative patient interface device 46

Patient interface device 46 includes mask 48, which in the illustrated embodiment is a nasal mask. However, any type of mask, such as a nasal/oral mask, a nasal cushion (e.g., including nasal prongs) or a full face mask, which facilitates the delivery of a flow of breathing gas to the airway of a patient, may be used as mask 48 while remaining within the scope of the present invention. Mask 48 includes cushion assembly 50 which is fluidly coupled to rigid support structure 52. Support structure 52 is fluidly coupled to elbow conduit 54. Elbow conduit 54 is structured to be coupled to delivery conduit 6 which is in fluid communication with pressure generating device 4. Support structure 52 includes a rigid shell and is structured to be coupled to a headgear assembly (not shown) for securing patient interface device 46 to the head of the patient.

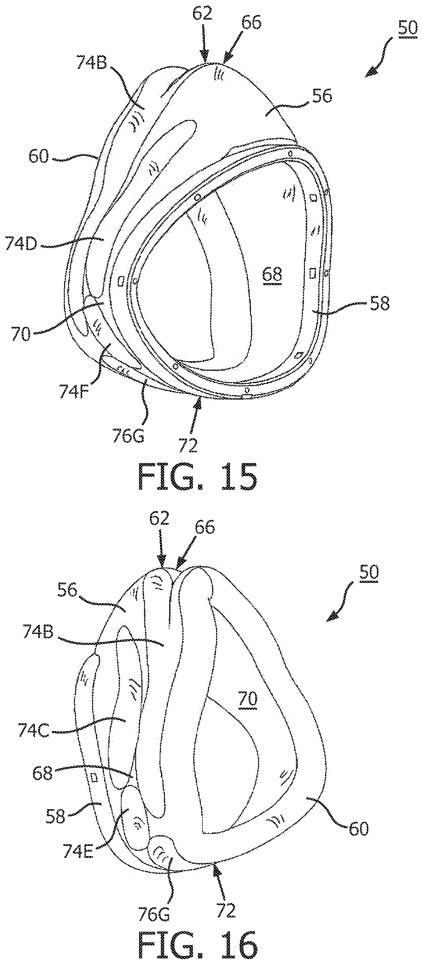

FIG. 15 is a front perspective view and FIG. 16 is a rear perspective view of cushion assembly 50 according to an alternative exemplary embodiment of the present invention. As seen in FIGS. 15 and 16, cushion assembly 50 includes sealing cushion 56 coupled to rigid frame 58. Rigid frame 58 facilitates attachment of cushion assembly 50 to support structure 52. Cushion 56 has a generally triangular-shaped periphery and is structured to fit over the patient's nose. However, it is well known in the art that various patient interfaces can be constructed which cover the patient's nose and mouth (nasal-oral), cover a majority of the patient's face (full-face mask), or fit within the patient's nares. The present invention contemplates that the unique aspects of this invention could be utilized on a variety of different patient interfaces without departing from the scope of this invention. Cushion 56 is configured to provide a flexible seal between the patient and patient interface device 46.

Cushion 56 has a patient contacting portion 60, a middle portion 62, and a connection portion 64. Each portion is specifically configured to achieve different goals. Patient contacting portion 60 is configured to provide a secure seal between the user (the patient) and patient interface device 46. Middle portion 62 is configured to provide support to cushion 56, and, as described in greater detail below, to provide for a controlled collapse, deformation and rebound of cushion 56 in response to forces exerted on cushion 56. Connection portion 64 is configured to provide a secure connection between cushion 56 and rigid frame 58. Furthermore, as noted above, cushion 56 has a generally triangular-shaped periphery and thus middle portion 62 includes apex wall 66, left side wall 68, right side wall 70 and bottom wall 72. In addition, as seen in FIGS. 15 and 16, middle portion 62 of cushion 56 includes a plurality of voids 74 provided in the inner side and/or outer side of one or more of apex wall 66, left side wall 68, right side wall 70 and bottom wall 72.

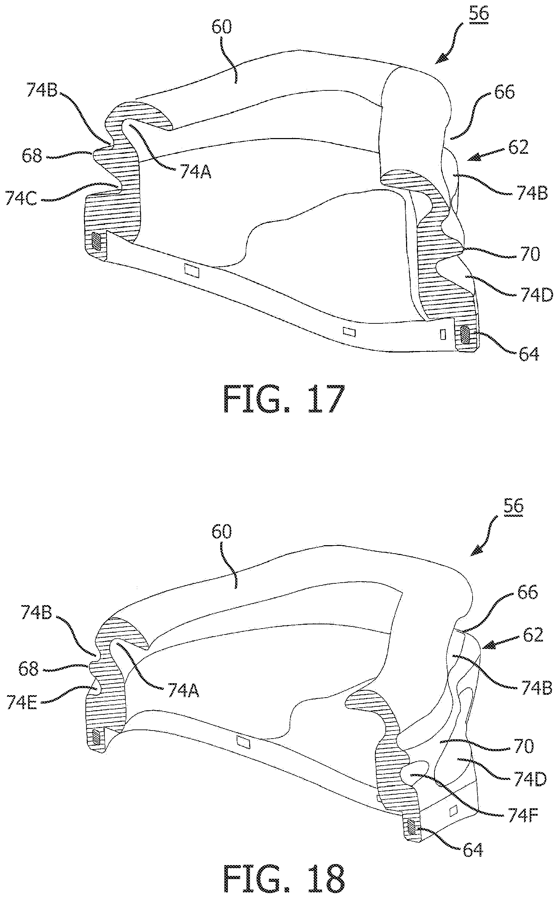

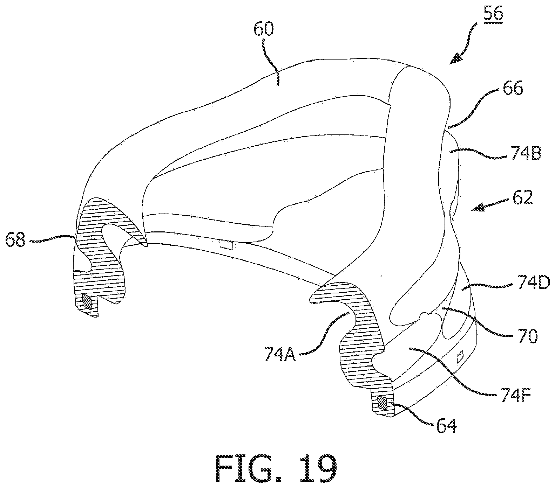

In the exemplary embodiment of cushion 56 shown in FIGS. 15 and 16, cushion 56 includes seven voids 74. FIGS. 17-19 are various cross-sectional views of cushion 56 which help to show the various voids 74 of the illustrated embodiment. As seen in FIGS. 17-19, those seven voids 74 include: (i) a first void 74A provided in the inner side (at the top thereof) of apex wall 66, left side wall 68, right side wall 70 and bottom wall 72 (void 74A thus extends continually around the entire inner top periphery of cushion 10), (ii) a second void 74B provided in the outer side of apex wall 66, left side wall 68 (at the top thereof), and right side wall 70 (at the top thereof) (void 74B thus extends continuously from the rear end of left side wall 68 to the rear end of right side wall 70), (iii) a third void 74C provided in the outer side of left side wall 68 (in the middle thereof), (iv) a fourth void 74D provided in the outer side of right side wall 70 (in the middle thereof), (v) a fifth void 74E provided in the outer side of left side wall 68 (at the rear, middle portion thereof), (vi) a sixth void 74F provided in the outer side of right side wall 70 (at the rear, middle portion thereof), and (vii) a seventh void 76G provided in the outer side of bottom wall 72.

In one particular, non-limiting exemplary embodiment of cushion 10, the dimensions of voids 74A-74G of cushion 56 are as follows. The depth dimensions provided are the proximal maximum depth or range of depths of the voids 26 and all dimensions are in mm. It should be understood that the values given are only some typical values which may change according to the compression force. Front is toward the tip of the triangular shape while base is the bottom. The width (W) of each void varies, and this width values gives are the proximal widths. Void 74A ranges in depth from 6 to 10 mm (from inner wall - extends all way round, W=12), void 74B ranges in depth from 3 to 6 mm (from outer wall - extends side to front 180 mm long, W=11), voids 74C and 74D 6 mm in depth (from outer wall - extends along side 60 mm long, W=11), voids 74E and 74F 5 mm in depth (from outer wall - extends along lower side 27 mm long, W=8.5), and void 74G 4.8 mm in depth (from outer wall - extends along base 79 mm long, W=9).

In addition, in one exemplary non-limiting embodiment, cushion 56, lime cushion 10, is defined from a unitary piece of soft, flexible, cushiony, elastomeric material, such as, without limitation, a two component silicone (RTV-2), an appropriately soft thermoplastic elastomer, a foam, or any combination of such materials, wherein the material has a hardness of between 5 and 60 on the Shore 00 scale.

In another exemplary non-limiting embodiment, which may or may not also use the material having the hardness just described, the portions of apex wall 66, left side wall 68, right side wall 70 and bottom wall 72 of middle portion 62 that do not include a void 74 have a cross-sectional thickness (shown by, for example, the arrows in FIG. 17) of 4 mm or greater. In an alternative exemplary embodiment, that cross-sectional thickness is 5 mm or greater, and in still another alternative exemplary embodiment, that cross-sectional thickness is between 4 mm and 10 mm or between 5 mm and 10 mm. These thicknesses are significantly greater than the wall thickness of current, prior art cushions, which typically have a thickness of between 0.3 and 1.5 mm.

Furthermore, as seen in FIGS. 17 and 18, in the illustrated embodiment, one or more cross-sectional portions of apex wall 66, left side wall 68, right side wall 70 and/or bottom wall 72 of middle portion 62 have a first void 74 on the outer side thereof and a second void 74 on the inner side thereof which are positioned so that they are aligned across from one another such that at least a portion of the first void 74 overlaps a portion of the second void 74 (with a section of the middle portion 62 being located therebetween). In other words, the overlapping first and second voids 74 are positioned such that at least a portion of the first void 74 and a portion of the second void 74 are located the same distance from a plane defining a bottom of middle portion 62. One example of such overlapping first and second voids 74 are voids 74A and 74B shown in FIGS. 17 and 18. In one particular, non-limiting embodiment, the amount of overlap between the voids 74 is 2 mm or more.

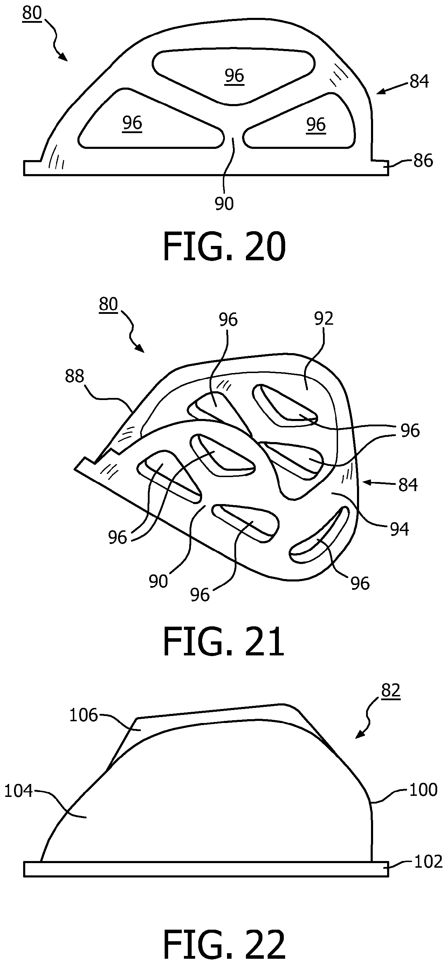

FIG. 20 is a side view and FIG. 21 is a front perspective view of cushion element 80 forming a part of a patient sealing assembly according an alternative exemplary embodiment of the present invention. Cushion element 80 has a generally triangular-shaped periphery and is structured to fit over the patient's nose. However, it is well known in the art that various patient interfaces can be constructed which cover the patient's nose and mouth (nasal-oral), cover a majority of the patient's face (full-face mask), or fit within the patient's nares. The present invention contemplates that the unique aspects of this invention could be utilized on a variety of different patient interfaces without departing from the scope of this invention. Cushion element 80, along with sealing flap assembly 82 (FIG. 22), described in greater detail below, is configured to form a patient sealing assembly portion of a patient interface device (e.g., having the general form of patient interface device 8 shown in FIG. 1) for providing a flexible seal between the patient and the patient interface device.

Cushion element 80 includes wall portion 84 and connection portion 86. Wall portion 84 is configured to provide support to cushion element 80 and to provide for a controlled collapse, deformation and rebound of cushion element 80 in response to forces exerted on the patient sealing assembly of which it forms a part. Connection portion 86 is configured to provide a secure connection between cushion element 80 and a support structure, such as an annular frame, of the patient interface device of which it forms a part. In the illustrated, non-limiting embodiment, cushion element 80 has a generally triangular-shaped periphery and thus wall portion 84 includes apex wall 88, left side wall 90, right side wall 92 and bottom wall 94.

In the exemplary embodiment, cushion element 80 is defined from a unitary piece of soft, flexible, cushiony, elastomeric material, such as, without limitation, a single component silicone rubber (RTV-1), a two component silicone (RTV-2), an appropriately soft thermoplastic elastomer, a foam, or any combination of such materials. In one particular, non-limiting embodiment, the material from which cushion element 80 is made has a hardness of between 5 and 60 on the Shore 00 scale. This material is much softer than materials that are used in current, prior art cushions, wherein the materials have a hardness of around 40 Shore A. In addition, as seen in FIGS. 20-21, wall portion 84 of cushion element 80 includes a plurality of orifices 96 extending completely through one or more of apex wall 88, left side wall 90, right side wall 92 and bottom wall 94.

It can thus be appreciated that the present invention contemplates providing voids that do not extend through the wall of the cushion (FIGS 1-19), providing orifices that extend all the way through the wall of the cushion (FIGS. 20-21). The present invention also contemplates providing both voids and orifices in/through the walls of the cushion. The specific arrangement, size, shape, geometry, location, depth, etc. of the voids and orifices can be selected to achieve a desired collapsing force or vector at a particular location along the perimeter of the cushion.

In another exemplary non-limiting embodiment, which may or may not also use the material having the hardness just described, the portions of apex wall 88, left side wall 90, right side wall 92 and bottom wall 94 of wall portion 84 that do not include an orifice 96 have a cross-sectional thickness of 3 mm or greater.

FIG. 22 is a side view of sealing flap assembly 82. As noted above, sealing flap assembly 82 is configured to be coupled to cushion element 80 to together form a patient sealing assembly portion of a patient interface device for providing a flexible seal between the patient and the patient interface device. In particular, sealing flap assembly 82 includes sealing flap portion 100 coupled to (e.g., by an overmolding process) rigid or semi-rigid frame 102, which is structured to enable sealing flap portion 100 to be attached to a support structure, such as an annular frame, of the patient interface device of which it forms a part. Sealing flap portion 100 is made from an elastomeric material, such as, without limitation, silicone, an appropriately soft thermoplastic elastomer, or any combination of such materials. Sealing flap portion 100 includes main body 104 and flap 106 and is much thinner than cushion element 100. In the exemplary embodiment, sealing flap portion has a cross-sectional thickness of between 0.1 to 0,5 mm.

As will be appreciated, because cushion element 80 includes orifices 96 that extend completely through wall portion 84, it is not air tight and, thus, alone cannot be used to deliver breathing gas to a patient. Instead, according to an aspect of an embodiment of the invention, sealing flap assembly 82 is placed over and generally surrounds cushion element 80 to cover and seal orifices 96 and together form an airtight patient sealing assembly that may be used in a patient interface device. In an attentive embodiment, sealing flap assembly may be configured to fit inside cushion element 80 to cover and seal orifices 96 and together form an airtight patient sealing assembly that may be used in a patient interface device.

With current masks and cushions, patients often develop red marks and sores on the face and complain about pressure points due to mask strapping pressure and cushion hardness. This primarily occurs on the upper lip, cheeks, and nose bridge area. Apertures 96 are advantageous as they allow cushion element 80 to collapse in one plane in specific areas during compression. This effect will lead to decreased pressure points and more even distribution of pressure. This effect will also require less strapping force of the patient interface device to the face of the patient to achieve a seal, all of which will increase patient comfort and therapy efficacy. Apertures 96 in cushion element 80 also provide areas with no material, which reduces the weight of cushion element 80 and the patient sealing assembly of which it forms a part on the patient's face. Excessive weight is a common complaint among patients.

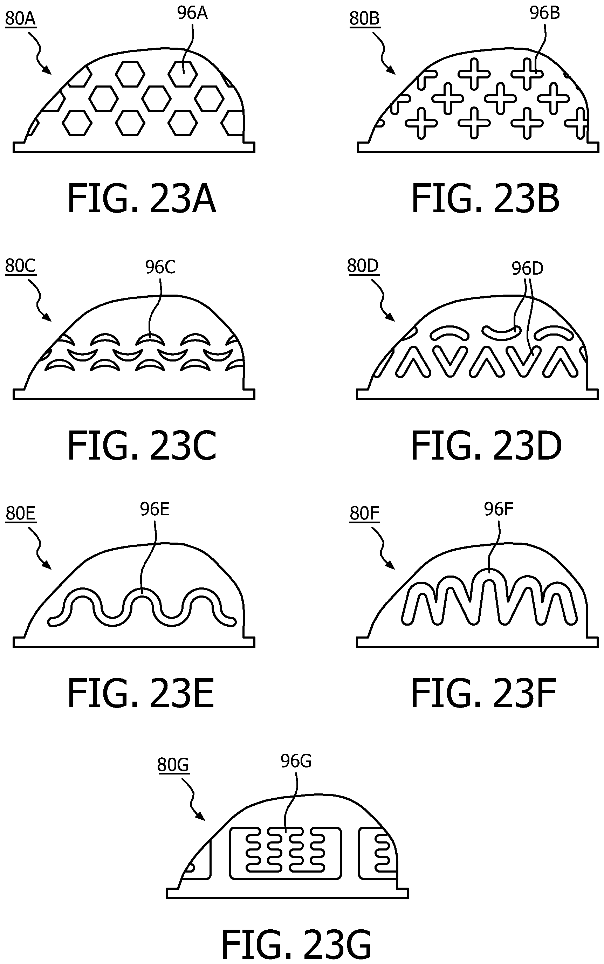

Furthermore, as seen in FIGS. 20-21, orifices 96 in the illustrated embodiment have a generally triangular shape. It should be understood, however, that orifices 96 may have any of a number of different shapes. The present invention further contemplates that voids 26, 74 can have any of a number of different shapes. FIGS. 23A-23G show alternative cushion elements 80A-80G wherein the orifices and/or voids have alternative shapes. In cushion element 80A, orifices and/or voids 96A have a hexagonal shape, in cushion element 80B, orifices and/or voids 96B have a plus sign shape, in cushion element 80C, orifices and/or voids 96C have a crescent shape, in cushion element 80D, orifices and/or voids 96D have a kidney bean and V shape, in cushion element 80E, orifice and/or voids 96E has a generally sinusoidal shape, in cushion element 80F, orifice and/or voids 96F has a repeating M shape, and in cushion element 80G, orifices and/or voids 96G have a an interlocking tower shape. Still other shapes are possible and all of the shapes shown can be used in any combination depending on the desired collapsing characteristics of the cushion.

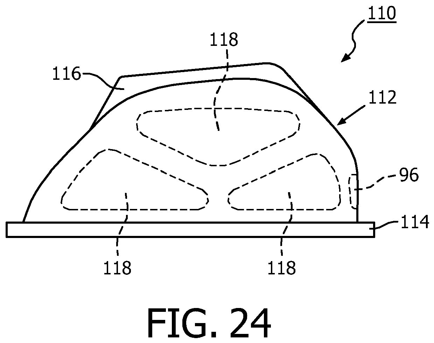

FIG. 24 is a side view of cushion 110 according a further alternative exemplary embodiment of the present invention. Cushion 110 has a generally triangular-shaped periphery and is structured to fit over the patient's nose. However, it is well known in the art that various patient interfaces can be constructed which cover the patient's nose and mouth (nasal-oral), cover a majority of the patient's face (full-face mask), or fit within the patient's nares. The present invention contemplates that the unique aspects of this invention could be utilized on a variety of different patient interfaces without departing from the scope of this invention. Cushion 110 is configured to alone form a patient sealing assembly portion of a patient interface device (e.g., having the general form of patient interface device 8 shown in FIG. 1) for providing a flexible seal between the patient and the patient interface device.

Cushion 110 includes wall portion 112, connection portion 114, and sealing flap 116. Wall portion 112 is configured to provide support to cushion 110 and to provide for a controlled collapse, deformation and rebound of cushion 110 in response to forces exerted on the patient sealing assembly of which it forms a part. Connection portion 114 is configured to provide a secure connection between cushion 110 and a support structure, such as an annular frame, of the patient interface device of which it forms a part. Flap portion 116 is configured to provide a secure seal between the user (the patient) and cushion 110. In the illustrated, non-limiting embodiment, cushion 110 has a generally triangular-shaped periphery and thus wall portion 112 includes an apex wall, a left side wall, a right side wall and bottom wall.

In the exemplary embodiment, cushion 110 is defined from a unitary piece of soft, flexible, cushiony, elastomeric material, such as, without limitation, a single component silicone rubber (RTV-1), a two component silicone (RTV-2), an appropriately soft thermoplastic elastomer, a foam, or any combination of such materials. In one particular, non-limiting embodiment, the material from which cushion 110 is made has a hardness of between 5 and 60 on the Shore 00 scale. This material is much softer than materials that are used in current, prior art cushions, wherein the materials have a hardness of around 40 Shore A. In addition, as seen in FIG. 24, wall portion 112 of cushion 110 includes a plurality of voids 118 on either or both of the inner and outer sides of wall portion 112. In the embodiment illustrated in FIG. 24, voids 118 are provided in the inner sides of wall portion 112, and thus are shown in dashed lines.

In the exemplary non-limiting embodiment, which may or may not also use the material having the hardness just described, the portions of wall portion 112 that do not include a void 118 have a cross-sectional thickness of 3 mm or greater, and the portions of wall portion 112 that define the bottom surface of each void 118 have a cross-sectional thickness of between 0.1 and 0.5 mm. Flap portion 116 also has a cross-sectional thickness of between 0.1 and 0.5 mm. In another embodiment, the portions of wall portion 112 that do not include a void 118 have a cross-sectional thickness T, and the portions of wall portion 112 that define the bottom surface of each void 118 have a cross-sectional thickness of 5% to 9% of T. In addition, in this embodiment, flap portion 116 has a cross-sectional thickness of between 5% to 9% of T.

Because voids 118 do not extend all the way through wall portion 112, cushion 110 may provide an air tight seal against the face of the patient, and therefore does not need to be used with sealing flap assembly 82. In addition, voids 118 in the illustrated embodiment have a generally triangular shape. It should be understood, however, that voids 118 may have any of a number of different shapes, such as those shown in FIGS. 23A-23G.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. A cushion (10, 30 56) for a patient interface device (8, 46), comprising:
a patient contacting portion (12, 32 60);
a connection portion (16, 36, 64) configured to provide a secure connection between the cushion and a support structure; and
a middle portion (14, 34 62) provided in between the patient contacting portion and the connection portion, the middle portion including one or more wall portions (18, 20, 22, 24, 34, 66, 68, 70, 72), wherein the one or more wall portions include a plurality of voids (26, 74) therein, wherein the patient contacting portion and the one or more wall portions are made of a material having a hardness of between 5 and 60 on the Shore 00 scale,
**characterized in that** the plurality of voids (26, 74) comprise at least one first void provided in an inner side of the one or more wall portions and at least one second void provided in an outer side of the one or more wall portions, and **in that** the parts of the one or more wall portions not having the plurality of voids (26, 74) provided therein all have a cross-sectional thickness of between 4 and 10 mm,
wherein the one or more wall portions comprise an apex wall (18, 66), a left side wall (20, 68), a right side wall (22, 70) and a bottom wall (24, 72), wherein the plurality of voids comprise: (i) a first void (26A, 74A) provided in an inner side of the apex wall, the left side wall, the right side wall and the bottom wall, (ii) a second void (26B, 74B) provided in an outer side of the apex wall, the left side wall, and the right side wall, (iii) a third void (26C, 76C) provided in the outer side of the left side wall below the second void, (iv) a fourth void (26D, 74D) provided in the outer side of the right side wall below the second void, (v) a fifth void (26E, 74E) provided in the outer side of the left side wall at least partially above the third void and below the second void, (vi) a sixth void (26F, 74F) provided in the outer side of the right side wall at least partially above the fourth void below the second void, and (vii) a seventh void (26G, 74G) provided in the outer side of the bottom wall,
wherein the at least one first void and the at least one second void are aligned across from one another such that at least a portion of the first void overlaps a portion of the second void with a section of the middle portion (14, 34 62) being located therebetween.

2. The cushion according to claim 1, wherein the patient contacting portion, the connection portion and the middle portion are made of a unitary piece of the material.

3. The cushion according to claim 2, wherein the material is a two component silicone (RTV-2).

4. The cushion according to claim 1, wherein the parts of the one or more wall portions not having the plurality of voids provided therein all have a cross-sectional thickness of between 5 mm and 10 mm.

5. The cushion according to claim 1, wherein the patient contacting portion and the one or more wall portions comprise a hollow bladder (38) filled with a material (40) having a hardness of between 5 and 60 on the Shore 00 scale.

6. The cushion according to claim 5, wherein the material is a gel, fluid or foam.

7. The cushion according to claim 5, wherein the bladder (38) is made of silicone, TPE or slush-molded rubber.

8. A patient interface device (8, 46) for delivering a flow of breathing gas to an airway of a patient, comprising:
a cushion (10, 30, 56) as claimed in any of claims 1 to 7.

9. A patient interface device (8, 46) according to claim 8, further comprising:
a support structure (7, 52); and
a coupling conduit (5, 54) coupled to the support structure.

## Patentansprüche

1. Ein Kissen (10, 30 56) für eine Patientenschnittstellenvorrichtung (8, 46), umfassend:
ein Patientenkontaktabschnitt (12, 32 60);
einen Verbindungsabschnitt (16, 36, 64) der so konfiguriert ist, dass er eine sichere Verbindung bereitstellt; und
einen mittleren Abschnitt (14, 34, 62), der zwischen dem Patientenkontaktabschnitt und dem Verbindungsabschnitt vorgesehen ist, wobei der mittlere Abschnitt einen oder mehrere Wandabschnitte aufweist (18, 20, 22, 24, 34, 66, 68, 70, 72), wobei der eine oder die mehreren Wandabschnitte eine Vielzahl von Hohlräumen (26, 74) umfassen, wobei der Patientenkontaktabschnitt und der eine oder die mehreren Wandabschnitte aus einem Material mit einer Härte zwischen 5 und 60 auf der Shore 00-Skala hergestellt sind,
die **dadurch gekennzeichnet sind, dass** die Mehrzahl von Hohlräumen (26, 74) mindestens einen ersten Hohlraum aufweist, der in einer Innenseite des einen oder der mehreren Wandabschnitte vorgesehen ist, und mindestens einen zweiten Hohlraum aufweist, der in einer Außenseite des einen oder der mehreren Wandabschnitte vorgesehen ist, Und dass die Teile des einen oder der mehreren Wandabschnitte, die nicht die Mehrzahl der darin vorgesehenen Hohlräumen (26, 74) aufweisen, alle eine Querschnittsdicke zwischen 4 und 10 mm aufweisen,
wobei die eine oder mehrere Wandabschnitte eine Scheitelwand (18, 66), eine linke Seitenwand (20, 68), eine rechte Seitenwand (22, 70) und eine Bodenwand (24, 72) umfassen, wobei die Mehrzahl von Hohlräume die folgenden umfassen: (i) einen ersten Hohlraum (26A, 74A), der in einer Innenseite der Scheitelwand, der linken Seitenwand, der rechten Seitenwand und der Bodenwand vorgesehen ist, (ii) einen zweiten Hohlraum (26B, 74B), der in einer Außenseite der Scheitelwand, der linken Seitenwand und der rechten Seitenwand vorgesehen ist, (iii) einen dritten Hohlraum (26C, 76C), der in der Außenseite der linken Seitenwand unterhalb des zweiten Hohlraums vorgesehen ist, (iv) einen vierten Hohlraum (26D, 74D), der in der Außenseite der rechten Seitenwand unterhalb des zweiten Hohlraums vorgesehen ist, (v) einen fünften Hohlraum (26E, 74E), der in der Außenseite der linken Seitenwand mindestens teilweise über dem dritten Hohlraum und unterhalb des zweiten Hohlraums vorgesehen ist, (vi) einen sechsten Hohlraum (26F, 74F), der in der Außenseite der rechten Seitenwand zumindest teilweise über dem vierten Hohlraum unterhalb des zweiten Hohlraums vorgesehen ist, und (vii) ein siebter Hohlraum (26G, 74G), der in der Außenseite der Bodenwand vorgesehen ist,
wobei der mindestens eine erste Hohlraum und der mindestens eine zweite Hohlraum sich so gegenüber ausgerichtet sind, dass zumindest ein Teil des ersten Hohlraums einen Abschnitt des zweiten Hohlraums mit einem Abschnitt des Mittelabschnitts (14, 34, 62) überlappt, der dazwischen liegt.

2. Das Kissen nach Anspruch 1, wobei der Patientenkontaktabschnitt, der Verbindungsabschnitt und der Mittelabschnitt aus einem Stück des Materials hergestellt sind.

3. Kissen nach Anspruch 2, wobei das Material ein Zweikomponenten-Silikon (RTV-2) ist.

4. Kissen nach Anspruch 1, wobei die Teile des einen oder der mehreren Wandabschnitte, die nicht die Mehrzahl der darin vorgesehenen Hohlräumen aufweisen, alle eine Querschnittsdicke zwischen 5 mm und 10 mm aufweisen.

5. Kissen nach Anspruch 1, wobei der Patientenkontaktabschnitt und der eine oder mehrere Wandabschnitte eine Hohlblase (38) umfassen, die mit einem Material (40) mit einer Härte zwischen 5 und 60 auf der Shore 00-Skala gefüllt ist.

6. Kissen nach Anspruch 5, wobei das Material ein Gel, eine Flüssigkeit oder ein Schaum ist.

7. Kissen nach Anspruch 5, wobei die Blase (38) aus Silikon, TPE oder aus Slush geformtem Gummi besteht.

8. Patientenschnittstellenvorrichtung (8, 46) zum Abgeben eines Atemgasflusses zu einem Atemweg eines Patienten, umfassend:
ein Kissen (10, 30, 56) nach einem der Ansprüche 1 bis 7.

9. Patientenschnittstellenvorrichtung (8, 46) nach Anspruch 8, ferner umfassend:
eine Stützstruktur (7, 52); und
eine mit der Tragstruktur verkoppelte Kopplungsleitung (5, 54).

## Revendications

1. Coussin (10, 30 56) pour un dispositif d'interface patient (8, 46) comprenant :
une partie en contact avec le patient (12, 32 60) ;
une partie de connexion (16, 36, 64) configurée pour fournir une connexion sûre entre le coussin et une structure de support ; et
une partie centrale (14, 34, 62) ménagée entre la partie en contact avec le patient et la partie de connexion, la partie centrale comprenant une ou plusieurs parties de paroi (18, 20, 22, 24, 34, 66, 68, 70, 72), dans laquelle la ou les parties de parois comprennent une pluralité de vides (26, 74) à l'intérieur, dans laquelle la partie en contact avec le patient et la ou les parties de paroi sont réalisées en un matériau ayant une dureté comprise entre 5 et 60 sur l'école Shore 00,
**caractérisé en ce que** la pluralité de vides (26, 74) comprend au moins un premier vide ménagé dans un côté intérieur de la ou des parties de paroi et au moins un second vide ménagé dans un côté extérieur de la ou des parties de paroi, et **en ce que** les parties de la ou des parties de paroi n'ayant pas la pluralité de vides (26, 74) ménagés à l'intérieur ont toutes une épaisseur en coupe transversale comprise entre 4 et 10 mm,
dans lequel la ou les parties de paroi comprennent une paroi de sommet (18, 66), une paroi latérale gauche (20, 68), une paroi latérale droite (22, 70) et une paroi inférieure (24, 72), dans lequel la pluralité de vides comprend : (i) un premier vide (26A, 74A) ménagé dans un côté intérieur de la paroi de sommet, la paroi latérale gauche, la paroi latérale droite et la paroi inférieure, (ii) un second vide (26B, 74B) ménagé dans un côté extérieur de la paroi de sommet, la paroi latérale gauche, et la paroi latérale droite, (iii) un troisième vide (26C, 76C) ménagé dans le côté extérieur de la paroi latérale gauche sous le second vide, (iv) un quatrième vide (26D, 74D), ménagé dans le côté extérieur de la paroi latérale droite sous le second vide, (v) un cinquième vide (26E, 74E) ménagé dans le côté extérieur de la paroi latérale gauche au moins partiellement au-dessus du troisième vide et sous le second vide, (vi) un sixième vide (26F, 74F) ménagé dans le côté extérieur de la paroi latérale droite au moins partiellement au-dessus du quatrième vide sous le second vide, et (vii) un septième vide (26G, 74G) ménagé dans la partie extérieure de la paroi inférieure,
dans lequel ledit au moins un premier vide et ledit au moins un second vide sont alignés l'un à travers l'autre de sorte qu'au moins une partie du premier vide chevauche une partie du second vide avec une section de la partie centrale (14, 34 62) située entre eux.

2. Coussin selon la revendication 1, dans lequel la partie en contact avec le patient, la partie de connexion et la partie centrale sont faites d'une pièce unique du matériau.

3. Coussin selon la revendication 2, dans lequel le matériau est un silicone à deux composants (RTV-2).

4. Coussin selon la revendication 1, dans lequel les pièces de la ou des parties de paroi n'ayant pas la pluralité de vides ménagés à l'intérieur ont toutes une épaisseur en coupe transversale comprise entre 5 mm et 10 mm.

5. Coussin selon la revendication 1, dans lequel la partie en contact avec le patient et la ou les parties de paroi comprennent une vessie creuse (38) remplie d'un matériau (40) ayant une dureté comprise entre 5 et 60 sur l'échelle Shore 00.

6. Coussin selon la revendication 5, dans lequel le matériau est un gel, un fluide ou une mousse.

7. Coussin selon la revendication 5, dans lequel la vessie (38) est faite de silicone, TPE ou caoutchouc coulé en coquille.

8. Dispositif d'interface patient (8, 46) pour fournir un flux de gaz de respiration aux voies aériennes d'un patient, comprenant :
un coussin (10, 30, 56) selon l'une quelconque des revendications 1 à 7.

9. Dispositif d'interface patient (8, 46) selon la revendication 8, comprenant en outre :
une structure de support (7, 52) et
un conduit de raccordement (5, 54) raccordé à la structure de support.
